# FASCICULE DE BREVET EUROPEEN

(11) **EP 2 370 810 B1**
(45) Date de publication et mention de la délivrance du brevet: **11.09.2019**
(21) Numéro de dépôt: 09801737.9
(22) Date de dépôt: 02.12.2009
(51) Int. Cl.: A61B 8/00, G01N 29/22

(54) **DISPOSITIF ET PROCÉDÉ D'ÉLASTOGRAPHIE**
ELASTOGRAPHIEVORRICHTUNG UND -VERFAHREN
ELASTOGRAPHY DEVICE AND METHOD

(30) Priorité: 04.12.2008 FR 0858278
(43) Date de publication de la demande: 05.10.2011
(73) Titulaire: Echosens, 75013 Paris (FR)
(72) Inventeur: SANDRIN, Laurent, F-94240 L'Hay les Roses (FR); BOSISIO, Matteo, F-75013 (FR); BASTARD, Cécile, F-75011 Paris (FR)
(74) Mandataire: Lebkiri, Alexandre
(86) Numéro de dépôt international: PCT/FR2009/052373
(87) Numéro de publication internationale: WO 2010/063951

(56) Documents cités:
- WO-A-2008/038184
- FR-A- 2 843 290
- FR-A- 2 869 521
- US-A1- 2005 165 291
- US-A1- 2007 080 275
- US-B1- 6 425 865
- BASTARD C ET AL: "Assessment of the elastic properties of heterogeneous tissues using transient elastography: Application to the liver" ULTRASONICS SYMPOSIUM, 2008. IUS 2008. IEEE, IEEE, PISCATAWAY, NJ, USA, 2 novembre 2008 (2008-11-02), pages 317-320, XP031443400 ISBN: 978-1-4244-2428-3

## Description

La présente invention concerne un dispositif et un procédé associé pour mesurer des propriétés viscoélastiques de tout milieu.

La présente invention se rapporte plus particulièrement à un dispositif et à un procédé d'élastographie de mesures quantitatives et/ou qualitatives de propriétés viscoélastiques, telles que l'élasticité et/ou la viscosité d'un tissu biologique humain ou animal. Plus précisément, un tel dispositif et un tel procédé sont adaptés pour mesurer les propriétés viscoélastiques de tissus biologiques appartenant aux animaux de laboratoire ou d'échantillon tissulaire.

Afin de mesurer les propriétés viscoélastiques tissulaires, il est connu d'utiliser l'élastographie impulsionnelle, comme décrit, par exemple, dans la demande de brevet numéro FR 2843290.

La demande de brevet FR 2869521 décrit un dispositif pour la mesure de l'élasticité d'un organe humain ou animal au travers d'un conduit, comportant un bras de liaison articulé, positionné entre un générateur de vibration et un transducteur ultrasonore, pour le maintien et positionnement dudit transducteur.

Ce document présente une mise en oeuvre d'un dispositif 10 selon l'art antérieur (figure 1). Ce dispositif 10 est composé d'une sonde 1 munie d'un générateur de vibration 2 générant une onde élastique basse fréquence dans un tissu, par exemple par vibration, et analysant la propagation de cette onde élastique basse fréquence à l'aide d'ondes ultrasonores haute fréquence émises et reçues par un transducteur ultrasonore 4 durant la propagation de l'onde élastique basse fréquence.

En outre, il est à noter que l'utilisateur 6 positionne ladite sonde 1 manuellement en vue d'assurer un contact permanent, entre le tissu et le transducteur ultrasonore 4. Ladite sonde 1 munie du générateur de vibration 2 et du transducteur ultrasonore 4 comporte également un déclencheur 5 commandant ces derniers. L'ensemble est relié à un calculateur 9.

Un tel dispositif est adapté pour mesurer les propriétés viscoélastiques d'organes de grande dimension situés au voisinage de l'épiderme contre lequel est positionnée la sonde 1 tel que par exemple le foie humain.

Un tel dispositif présente cependant des inconvénients. Notamment, il n'est pas adapté aux organes de petite dimension. En effet, la sonde engendre une compression des tissus lorsqu'elle est apposée voire positionnée au voisinage ou au contact de l'organe (plus le volume du tissu est petit et plus le phénomène de compression est important).

En outre, un tel dispositif n'est pas adapté aux organes de petite dimension car la taille du transducteur ultrasonore est trop importante et le positionnement de la sonde est approximatif et aléatoire.

A ce titre, on peut citer la souris, dont la surface de l'épiderme donnant accès au foie est de l'ordre de 1 cm². Cette contrainte dimensionnelle ne permet pas à l'utilisateur d'assurer un positionnement optimal qui dépend non seulement de la taille du tissu mais également de la variabilité inter-individus et/ou interespèces.

Un inconvénient supplémentaire réside dans la nécessité pour l'utilisateur de maintenir manuellement d'une part, un contact permanent et satisfaisant entre le transducteur ultrasonore et l'épiderme afin que les ultrasons puissent se propager et, d'autre part, une perpendicularité optimale.

Or, pour déclencher des ondes élastiques basse fréquence, l'utilisateur appuie sur le déclencheur 5 ce qui a pour effet de générer des variations du contact de la sonde avec l'épiderme faussant la perpendicularité et, de facto, entraînant une surestimation des mesures proche du transducteur ultrasonore.

En outre, un tel mouvement pose des problèmes de repétabilité des mesures. En effet, dès lors, que la position du transducteur ultrasonore a été modifiée, il est impossible de positionner à nouveau le transducteur ultrasonore précisément à l'endroit où les premières mesures ont été effectuées. Ceci est très handicapant pour des mesures effectuées dans des tissus hétérogènes, tel que par exemple, le rein.

Par ailleurs, une surestimation de l'élasticité mesurée due entre autre, à un phénomène de diffraction dans le champ proche de la source de génération d'ondes basse fréquence se produit sur une épaisseur qui dépend notamment, des dimensions de cette source, des propriétés viscoélastiques, du milieu, de l'épaisseur de la couche adipeuse ainsi que de la fréquence de l'onde élastique basse fréquence.

Cette épaisseur, en deçà de laquelle les mesures sont impossibles est de l'ordre de 20 mm pour une fréquence centrale f de l'onde élastique basse fréquence de 50 Hz. Pour palier ce problème, il est fait abstraction des données sur cette épaisseur, à savoir 20 mm, dans le calcul des propriétés viscoélastiques interdisant de ce fait des mesures sur une épaisseur inférieur à 20 mm.

Dans ce contexte l'invention vise à proposer un dispositif non invasif permettant de s'affranchir des problèmes précités en proposant un dispositif permettant un maintien et un positionnement précis, perpendiculaire et au contact du tissu, d'un transducteur ultrasonore pour déterminer les propriétés viscoélastiques de celui-ci.

Un tel dispositif, est avantageux également par sa conception, qui même si elle requiert des connaissances accrues et de nombreux développements, propose un dispositif simple d'utilisation, non invasif et peu coûteux.

A cette fin, l'invention propose un dispositif d'élastographie de mesures quantitatives et/ou qualitatives de propriétés viscoélastiques de tout milieu, ledit dispositif comportant :
- un générateur de vibration pour la génération d'au moins une onde élastique basse fréquence ;
- au moins un transducteur ultrasonore pour observer la propagation de ladite au moins une onde élastique basse fréquence ;
- un déclencheur pour déclencher au moins une mesure ;
ladite au moins une mesure comportant les étapes suivantes :
- générer au moins une onde élastique basse fréquence dans un milieu ;
- émettre et acquérir simultanément à la génération d'au moins une onde élastique basse fréquence des ultrasons haute fréquence à l'aide dudit transducteur ultrasonore pour observer la propagation de ladite au moins une onde élastique basse fréquence ;
ledit dispositif comportant aussi un tube déformable, positionné entre ledit générateur de vibration et ledit transducteur ultrasonore, pour un maintien et un positionnement dudit transducteur ultrasonore situé à l'extrémité dudit tube déformable de façon à assurer, pendant au moins une mesure, la perpendicularité et le contact entre ledit transducteur et le milieu; et
au moins une tige située à l'intérieur du tube déformable, apte à transmettre les ondes élastiques basse fréquence dudit générateur de vibration audit transducteur ultrasonore.

On entend par tube déformable, un tube possédant une liberté de mouvement dans plusieurs directions, sans aucune contrainte angulaire, et maintenant sa position lorsqu'aucun effort ne lui est appliqué en en vue de le mouvoir.

Le milieu est désigné indifféremment par le terme tissu.

Outre les caractéristiques principales qui viennent d'être mentionnées dans le paragraphe précédent, le dispositif selon l'invention peut présenter une ou plusieurs caractéristiques supplémentaires ci-dessous, considérées individuellement ou selon toutes les combinaisons techniquement possibles :
- ledit dispositif d'élastographie comporte un support pour maintenir spatialement le transducteur ultrasonore ;
- ledit dispositif d'élastographie comporte un système ressort situé entre ledit transducteur ultrasonore et ledit tube déformable ;
- ledit dispositif comporte un tube de préhension ;
- ledit générateur de vibration est formé par une table vibrante ;
- ledit générateur de vibration est formé par un dispositif annexe de type haut-parleur ;
- ledit dispositif d'élastographie comprend un système de réglage de précision pour ajuster en rotation et/ou en translation la position dudit transducteur ultrasonore ;
- ledit générateur de vibration se situe à l'extrémité opposée du tube déformable où se situe le transducteur ultrasonore ;
- ledit dispositif comporte une barrette ultrasonore d'imagerie haute fréquence ;
- ledit dispositif est apte à être couplé avec un dispositif de synchronisation organique ;
- ledit transducteur ultrasonore possède un diamètre actif inférieur à 3 mm.

La présente invention a également pour objet un procédé d'élastographie de mesures quantitatives et/ou qualitatives de propriétés viscoélastiques de tout milieu caractérisé en ce qu'il met en oeuvre un dispositif conforme à l'invention; ledit procédé comprend au moins les étapes suivantes:
- positionner, en déformant ledit tube déformable, perpendiculairement et au contact du milieu ledit au moins un transducteur ultrasonore ;
- ajuster la position dudit transducteur ultrasonore en translation et/ou en rotation ;
- déclencher au moins une mesure ;
- maintenir en position et au contact ledit transducteur ultrasonore pendant au moins une mesure ;
ladite mesure comprenant les étapes suivantes :
- générer au moins une onde élastique basse fréquence ;
- émettre et acquérir simultanément à la génération d'au moins une onde élastique basse fréquence des ultrasons haute fréquence à l'aide dudit transducteur ultrasonore pour observer la propagation de ladite au moins une onde élastique basse fréquence ;
- calculer au moins un paramètre relatif aux propriétés viscoélastiques du milieu tel que les variations des déplacements et/ou des déformations et/ou des vitesses de déplacement et/ou des vitesses de déformations engendrés dans le milieu ou plus généralement tout paramètre relatif aux propriétés viscoélastiques ;
- calculer les propriétés viscoélastiques du milieu.

Une caractéristique avantageuse supplémentaire réside en ce que ledit transducteur ultrasonore est maintenu, par ledit tube déformable, en position et au contact du milieu pendant une série de mesures.

Avantageusement les ondes élastiques basse fréquence sont déclenchées par l'utilisateur de façon aléatoire débutant de façon synchronisée avec un rythme respiratoire et/ou cardiaque.

De préférence, l'émission ultrasonore haute fréquence est réalisée sur une gamme de fréquence comprise entre 8 MHz et 100 MHz, et de préférence entre 10 MHz et 50 MHz.

De même, les ondes élastiques basse fréquence sont générées sur une gamme de fréquence comprise entre 50 Hz et 10000 Hz, lesdites ondes élastiques basse fréquence pouvant être de l'ordre de 400 Hz.

En outre, ladite au moins une onde élastique basse fréquence est générée par vibration mécanique et/ou par compression mécanique et/ou par focalisation ultrasonore.

D'autres caractéristiques et avantages de l'invention ressortiront clairement de la description qui en est donnée ci-dessous, à titre indicatif et nullement limitatif, de modes de réalisations faisant références aux figures annexées ci-jointes sur lesquelles :
- La figure 1 est une représentation schématique simplifiée d'un dispositif 10 selon l'art antérieur.
- La figure 2 est une représentation schématique simplifiée d'un dispositif selon l'invention pour assurer un positionnement d'au moins un transducteur ultrasonore.
- La figure 3 est une représentation schématique simplifiée d'un dispositif selon l'invention muni d'un tube de préhension.
- La figure 4 est une représentation schématique simplifiée d'un dispositif selon l'invention muni d'un support de type étau.
- La figure 5 est une représentation schématique simplifiée d'une barrette ultrasonore dédiée à d'imagerie pouvant faire partie du dispositif d'élastographie.

Pour des raisons de clarté, les mêmes éléments ont été désignés par des références similaires. De même, seuls les éléments utiles pour la compréhension de l'invention ont été représentés de manière schématique et ceci sans respect de l'échelle.

Pour la suite de la description, on entend par élastographie une technique de mesure des propriétés viscoélastiques dans laquelle un générateur de vibration génère, par contact direct ou indirect avec un tissu, une ou plusieurs ondes élastiques basse fréquence se propageant dans ce tissu.

La forme temporelle de cette onde élastique basse fréquence peut être arbitraire, mais plus généralement de type impulsionnelle, transitoire ou périodique (entretenue, monochromatique).

Cette vibration est en général obtenue de manière mécanique mais peut également être obtenue par pression de radiation, par hyperthermie ultrasonore ou par des vibrations internes du corps (battements cardiaques, pouls, etc.).

En outre, différents types de transducteurs ultrasonores monoélément ou multiélément peuvent être utilisés. Il peut s'agir par exemple, et de manière non limitative, d'un transducteur de type couronne, annulaire, matrice 2D, barrette linéaire ou convexe, d'un transducteur monoélément, d'un transducteur triélément, d'un transducteur de type étoilé, etc...

De plus, on entend par animaux de laboratoire, de manière non limitative, notamment, les amphibiens, les poissons, les reptiles ainsi que les mammifères, tel que par exemple la souris, le rat, le lapin, le chat, le chien et le singe.

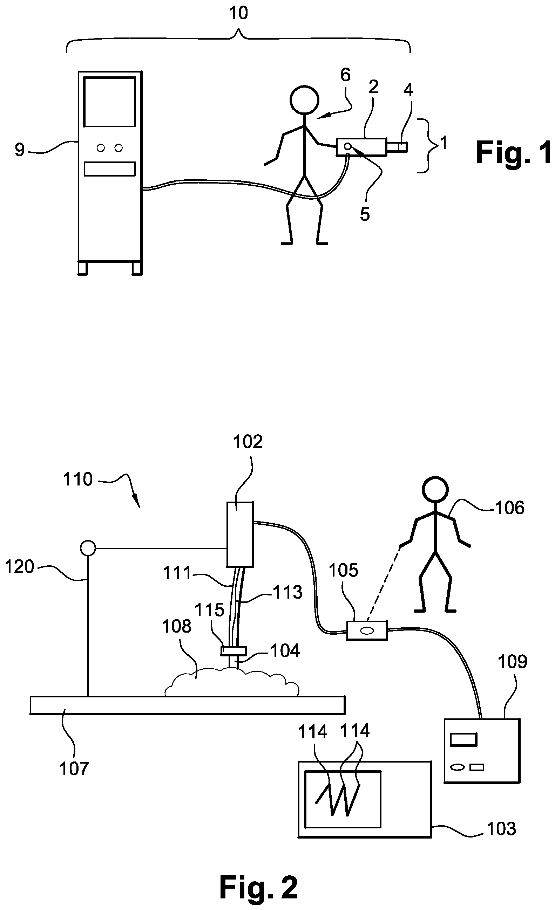

La figure 1 présente les dispositifs de l'art antérieur.

Sur la Figure 2, sont représentés, un utilisateur 106, un dispositif de synchronisation organique 103, une table 107, un animal de laboratoire 108 et un dispositif 110 d'élastographie selon l'invention, le dispositif 110 selon l'invention comporte :
- un tube déformable 111 ;
- au moins un transducteur ultrasonore 104 ;
- un support 120 ;
- un générateur de vibration 102 ;
- un déclencheur 105 ;
- un fil coaxial 113 pour la transmission et la réception des signaux ultrasonores ;
- un système ressort 115 ;
- un système 109 d'analyse, de traitement et de calcul.

Le tube déformable 111 assure le positionnement, le maintien d'au moins un transducteur ultrasonore 104 et assure la transmission d'ondes élastiques basse fréquence. Plus particulièrement, ce tube 111 maintien sa position lorsqu'il à été déformé

Le support 120 assure le maintien :
- du tube déformable 111 ;
- du générateur de vibration 102 pour la génération des ondes élastiques basse fréquence ;
- d'au moins un transducteur ultrasonore 104 pour l'émission et l'acquisition ultrasonore.

Le support 120 permet de maintenir les différents éléments précités sans requérir une quelconque intervention de l'utilisateur 106.

Le déclencheur 105 permet le déclenchement des mesures.

Le système ressort 115 situé, par exemple, entre l'extrémité du tube déformable 111 et le transducteur ultrasonore 104 permet de compenser l'effort exercé sur le tissu lors de la mise en contact du transducteur ultrasonore 104 avec ledit tissu. De ce fait, aucun effort de compression n'est engendré sur le tissu. Ceci permet d'éviter toute modification des propriétés viscoélastiques du tissu.

Nous allons décrire dans ce qui suit le fonctionnement du dispositif 110 selon l'invention.

La première étape consiste à positionner le transducteur ultrasonore 104 au contact du tissu à mesurer ou au contact du tissu avoisinant ledit tissu.

Pour ce faire, l'utilisateur 106 déforme le tube déformable 111 maintenu par le support 120 de façon à approcher au plus proche le transducteur ultrasonore 104 du tissu.

Ce tube déformable 111 est avantageux par sa structure qui permet, d'une part, de mouvoir le transducteur ultrasonore 104 aisément dans l'espace et ce sans aucune contrainte angulaire et d'autre part, de maintenir la position choisie sans que le tube déformable 111 se déforme du fait de sa ductilité.

Ainsi, le tube déformable 111 permet d'ajuster la position du transducteur ultrasonore 104 de façon à respecter la perpendicularité et le contact dudit transducteur ultrasonore 104 avec le tissu à mesurer.

La structure du tube déformable 111 peut être formé par:
- de multiples éléments reliés entre eux par une liaison rotule ; en d'autres termes, le tube déformable 111 est dans ce cas formé par un tube articulé ;
- un tube déformable élastiquement ; ou
- de façon plus générale, tout autre type de tube pouvant :
   - conserver sa forme lorsqu'aucun effort ne lui est appliqué ; et
   - modifier sa forme dès lors qu'un effort lui est appliqué.

De plus, selon une possibilité offerte par l'invention le dispositif est couplé avec un système de navigation chirurgicale, non représenté, consistant à :
- premièrement, localiser la zone d'intérêt à mesurer à l'aide d'un dispositif d'imagerie médicale, du type, imagerie par rayon-x, imagerie par résonance magnétique ou tout autre type d'imagerie autorisant la visualisation des tissus biologiques ;
- deuxièmement, positionner le transducteur ultrasonore 104 de façon électromécanique, électro-hydraulique, électropneumatique ou manuelle.

Un tel positionnement permet alors à l'utilisateur 106 de réaliser des mesures de propriétés viscoplastiques.

Lesdites mesures consistant à :
- générer au moins une onde élastique basse fréquence par l'intermédiaire du générateur de vibration 102 ;
- émettre et acquérir simultanément à la génération d'au moins une onde élastique basse fréquence des ultrasons haute fréquence à l'aide dudit transducteur ultrasonore 104 pour observer la propagation de ladite au moins une onde élastique basse fréquence.

Pour ce faire, le transducteur ultrasonore 104 doit tout au long des mesures:
- rester au contact du tissu pour ne pas perdre le signal ultrasonore et pour effectuer au moins une série de mesures au même endroit. Ceci est assuré par la structure spécifique du tube déformable 111 lui permettant de conserver sa forme.
- être positionné de façon perpendiculaire pour éviter une surestimation des mesures. Cette caractéristique étant réalisée par la structure déformable libre de contrainte angulaire du tube déformable 111.

Particulièrement, les organes de petite dimension ou les organes dont l'accessibilité est réduite requièrent une telle précision de positionnement.

Cette difficulté d'accessibilité est fréquemment rencontrée chez les petits animaux de laboratoire tels que les souris, par exemple, la souris sauvage (wild type, en anglais) adulte de longueur généralement 8 cm possède un foie dont le volume est d'environ 1.5 cm³, cependant la majeure partie de celui-ci est situé derrière la cage thoracique, obligeant ainsi, l'utilisateur 106 à effectuer des mesures sur une surface de l'épiderme comprise entre 10 mm² et 1 cm² et plus précisément sur une profondeur généralement comprise entre 1 mm et 20 mm sous le derme et de préférence 2 à 6 mm.

A cet effet, lors de la génération d'au moins une onde élastique basse fréquence, le générateur de vibration 102 transmet la vibration au transducteur ultrasonore 104 via le tube déformable 111. Comme représenté sur les figures 2, 3 et 4, le générateur de vibration 102 est séparé du transducteur ultrasonore 104 par le tube déformable 111. Le transducteur ultrasonore 104 possède un diamètre actif inférieur ou égal à 3 mm. Ce petit diamètre permet, d'une part, le positionnement du transducteur ultrasonore 104 sur une petite surface et, d'autre part, de s'affranchir de la zone de diffraction rencontrée par les dispositifs selon l'art antérieur.

En sus, effectuer des mesures sur une profondeur inférieur à 20 mm nécessite de générer des ondes élastiques basse fréquence possédant une fréquence centrale f comprise entre 50 et 10000 Hz et de préférence entre 100 et 3000 Hz, cette fréquence pouvant être typiquement de l'ordre de 400 Hz.

Puis, afin d'observer la propagation des ondes élastiques basse fréquence on utilise le transducteur ultrasonore 104 de type monoélément, ou multiélément adapté, pour générer des ultrasons haute fréquence compris par exemple entre 8 et 100 Mhz et de préférence compris entre 10 et 50 Mhz.

De telles mesures sont initialisées par l'utilisateur 106 par l'intermédiaire du déclencheur 105, lequel déclencheur est relié au générateur de vibration 102 et au transducteur ultrasonore 104 par un fil, par réseaux informatique de type WIFI ou tout autre dispositif autorisant le transfert des données.

Ainsi, le générateur de vibration 102 génère au moins une onde élastique basse fréquence, laquelle peut être transmise par le transducteur ultrasonore 104 lui-même ou par un dispositif annexe non représenté, tel que par exemple un haut-parleur.

En outre, on notera que le rythme cardiaque ainsi que le rythme respiratoire génèrent naturellement des ondes élastiques basse fréquence de type monochromatique. Dans le tissu se trouvent donc trois type d'ondes élastiques basse fréquence interférant entre elles, typiquement ces ondes peuvent être générées par :
- le dispositif selon l'invention ;
- le système cardiaque ;
- le système respiratoire.

Les dispositifs selon l'art antérieur font abstraction des interférences générées par le rythme respiratoire et/ou cardiaque. Pourtant, ces phénomènes biologiques engendrent des déplacements du référentiel altérant fortement la mesure des propriétés viscoélastiques ou la rendant impossible.

Au cours d'une inspiration, l'air pénètre dans les poumons, lesquels augmentent de volume, puis inversement, lors de l'expiration les poumons diminuent de volume. Ce phénomène, de gonflement et dégonflement peut engendrer de grands déplacements et ainsi fausser les mesures.

De ce fait, un dispositif d'élastographie selon l'art antérieur restreint le domaine d'utilisation. Cette incapacité de mesure est considérable pour les petits animaux car si chez l'homme ce rythme respiratoire est assez faible, à savoir 16 pulses par minute ce rythme respiratoire est au contraire très élevé chez les animaux de laboratoire. Plus précisément, tandis que le lapin approche les 60 pulses par minute, la souris avoisine les 220 pulses par minute.

Avantageusement, le dispositif 110 selon l'invention offre la possibilité de synchroniser la génération des ondes élastiques basse fréquence avec la fréquence biologique à l'aide d'un électrocardiogramme et/ou d'un synchro pulmonaire 103 plus communément connu sous le nom de synchronisation organique.

En effet, le cycle respiratoire est composé alternativement d'une inspiration pendant laquelle l'air pénètre dans les poumons puis inversement d'une expiration durant laquelle l'air est expulsé desdits poumons engendrant de forts déplacements tissulaires impliquant et des extensions et compressions tissulaires.

Il est donc important de déclencher les mesures à un temps t déterminé de façon répétée pour que chaque mesure ou série de mesures obtenues soit représentative d'une zone d'intérêt similaire.

A cet effet, le dispositif selon l'invention peut être couplé avec un dispositif de synchronisation organique 103 permettant de synchroniser les mesures avec le rythme respiratoire.

Cette mise en oeuvre est nécessaire et avantageuse chez les souris dont le foie comporte 6 lobes (lobe médian, lobe gauche, 2 lobes droits et 2 lobes caudales), lequel foie peut comporter non seulement une hétérogénéité organique mais également tissulaire obligeant l'utilisateur 106 à réaliser les mesures à un endroit précis pour au moins une série de mesures.

En référence à la figure 2 l'utilisateur 106 peut par exemple déclencher une série de mesures synchronisées avec un système de synchronisation organique 103 adapté pour déclencher des ondes élastiques basse fréquence à chaque pic 114 représentatif du front d'onde maximum correspondant à une inspiration maximum.

Ainsi, les mesures sont déclenchées par l'utilisateur mais ne débutent qu'au moment où le pic 114 représentatif du front d'onde maximal est atteint.

Puis, pour observer la propagation de ces ondes élastique basse fréquence, des ondes ultrasonores haute fréquence sont émises et acquises de manière simultanée par le transducteur ultrasonore 104.

Ces ondes ultrasonores haute fréquence sont généralement comprises entre 8 et 100 MHz et comprises de préférence entre 10 et 50 MHz.

Ces données sont ensuite transmises à un système 109 d'analyse, de traitement de donnée et de calcul, informant en temps réel l'utilisateur 106 sur les propriétés viscoélastiques de la zone d'intérêt.

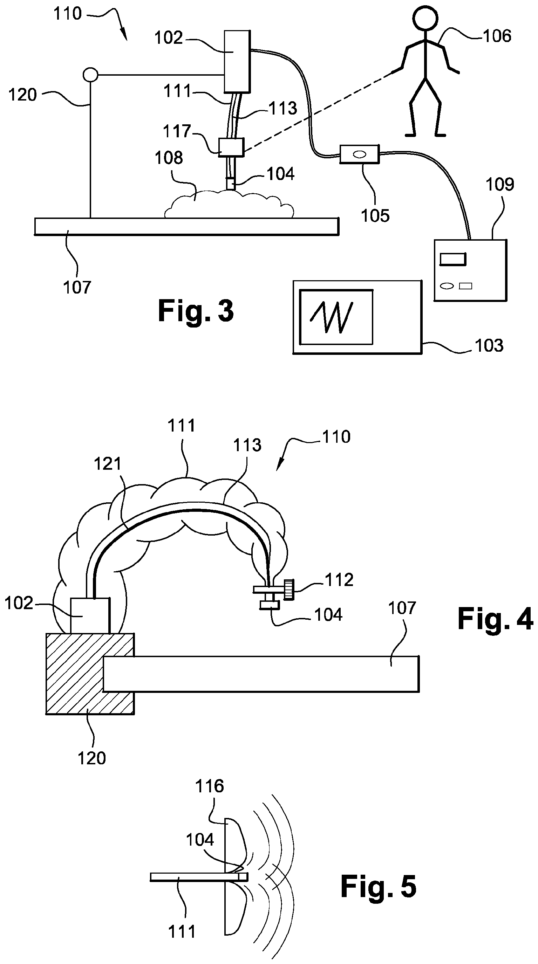

Selon une variante conforme à l'invention représentée figure 3, le dispositif d'élastographie 110 est muni d'un tube de préhension 117.

Ainsi, l'excitation basse fréquence générée par le générateur de vibration 102 est transmise au transducteur ultrasonore 104 via le tube déformable 111 situé à l'intérieur du tube de préhension 117.

Une telle mise en oeuvre est avantageuse par sa maniabilité, puisque l'utilisateur 106 positionne manuellement le transducteur ultrasonore 104 sans pour autant altérer la transmission des ondes élastiques basse fréquence effectuée par le tube déformable 111, du générateur de vibration 102 au transducteur ultrasonore 104. Ce dernier faisant, outre sa fonction d'émission et d'acquisition ultrasonore, office de palpeur.

En outre, le support 120 peut être de type étau tel qu'illustré sur la figure 4.

L'encombrement minimum de ce support facilite la maniabilité et le transport du dispositif d'élastographie 110.

Comme illustré figure 4, le dispositif d'élastographie peut comporter un système de réglage de précision 112, pour ajuster en rotation et/ou en translation la position du transducteur ultrasonore 104 sans avoir besoin de déformer le tube déformable 111.

Selon l'invention les ondes élastiques basse fréquence sont transmises au palpeur 104 via une tige 121 située à l'intérieur du tube déformable 111.

En outre, selon une variante supplémentaire d'un dispositif 110 conforme à l'invention représenté de façon schématique figure 5, le dispositif 110 est muni d'une barrette ultrasonore d'imagerie 116, de type échographe, permettant à l'utilisateur de s'assurer que le transducteur ultrasonore 104 est positionné dans l'axe de la zone d'intérêt.

Pour ce faire, le dispositif selon l'invention peut comprendre au moins une barrette ultrasonore d'imagerie 116 et au moins un transducteur ultrasonore 104 dédié à l'élastographie.

Le transducteur ultrasonore 104 se déplace selon une direction perpendiculaire ou sensiblement perpendiculaire au plan barrette d'imagerie de façon à transmettre dans le milieu au moins une onde élastique basse fréquence en provenance du générateur de vibration 102. Selon une variante non représentée le transducteur ultrasonore peut se déplacer selon l'axe x, l'axe y, et/ou l'axe z et/ou en rotation.

La barrette échographique 116, haute fréquence, informe l'utilisateur de la situation spatiale et des mouvements des organes, lui permettant ainsi de positionner le palpeur 104 dans l'axe dudit organe d'intérêt. Ainsi, le transducteur ultrasonore 104 situé à l'extrémité du tube déformable 111 et en contact avec l'épiderme est utilisé pour observer la propagation des ondes ultrasonores basse fréquence informant des propriétés viscoélastiques du tissu biologique.

En sus, comme illustré sur la figure 5, la barrette ultrasonore 116 haute fréquence possède une forme dédiée dont l'objectif est de recouvrir une zone optimale. A cet effet, ladite forme peut être, bombée, circulaire, ovale, rectangulaire, triangulaire ou tout autre forme géométrique aboutissant au même résultat, à savoir le recouvrement d'une zone par les ultrasons haute fréquence.

Les étapes éventuelles de mise en oeuvre d'un dispositif selon l'invention illustrée figure 5 consistent notamment à :
- imager le tissu avec une barrette ultrasonore 116 d'imagerie haute fréquence pour déterminer la situation spatiale de la zone d'intérêt ;
- positionner le transducteur ultrasonore 104 dans l'axe de la zone d'intérêt à l'aide du tube déformable 111,
- générer au moins une onde élastique basse fréquence,
- émettre et acquérir simultanément des ultrasons haute fréquence à l'aide du transducteur d'élastographie 104 pour mesurer les propriétés viscoélastiques.

Selon une variante avantageuse de l'invention, non illustrée ladite barrette ultrasonore 116 haute fréquence est composée de multiples éléments, lesquels peuvent être utilisés d'une part pour imager le milieu et d'autre part pour générer au moins une onde élastique basse fréquence.

La barrette peut générer au moins une onde élastique basse fréquence dans le milieu de façon :
- mécanique, par déplacement d'au moins un élément ;
- mécanique, par déplacement de ladite barrette ;
- électronique, par focalisation ultrasonore.

Selon une variante supplémentaire offerte par l'invention, le générateur de vibration peut être formé par la table 107 sur laquelle repose le tissu à mesurer. Dans une telle mise en oeuvre l'utilisateur 106 fait vibrer la table 107 puis suit le déplacement des ondes ultrasonores générées par ladite table 107 au moyen d'émission et acquisition ultrasonore du transducteur ultrasonore 104.

On notera que le domaine d'application de l'invention concerne les animaux de laboratoire. Une telle application peut bien entendu être étendue à l'industrie pharmaceutique, alimentaire, chimique, au domaine médical pour déterminer les propriétés viscoélastiques d'échantillon tissulaire humain ou animal ou d'échantillon issu de cultures cellulaire invivo, ex-vivo, in vitro.

L'invention est décrite dans ce qui précède à titre d'exemple. Il est entendu que l'homme du métier est à même de réaliser différentes variantes du dispositif et du procédé pour la mesure de l'élasticité d'un tissu humain ou animal, en particulier concernant la disposition ou l'agencement des différents éléments constituant ledit dispositif ou l'ordre ainsi que l'importance des étapes dudit procédé sans pour autant sortir du cadre du brevet.

## Revendications

1. Dispositif (110) d'élastographie de mesures quantitatives et/ou qualitatives de propriétés viscoélastiques de tout milieu, ledit dispositif (110) comportant :
- un générateur de vibration (102) pour la génération d'au moins une onde élastique basse fréquence ;
- un transducteur ultrasonore (104) pour observer la propagation de ladite au moins une onde élastique basse fréquence ;
- un déclencheur (105) pour déclencher au moins une mesure ;
ladite au moins une mesure comportant les étapes suivantes :
- générer au moins une onde élastique basse fréquence dans un milieu ;
- émettre et acquérir simultanément à la génération d'au moins une onde élastique basse fréquence des ultrasons haute fréquence à l'aide dudit transducteur ultrasonore (104) pour observer la propagation de ladite au moins une onde élastique basse fréquence ;
ledit dispositif comportant aussi:
• un tube déformable (111), positionné entre ledit générateur de vibration (102) et ledit transducteur ultrasonore (104), pour un maintien et un positionnement dudit transducteur ultrasonore (104) situé à l'extrémité dudit tube déformable (111) de façon à assurer, pendant au moins une mesure, la perpendicularité et le contact entre ledit transducteur ultrasonore (104) et le milieu ;
• au moins une tige (121) située à l'intérieur du tube déformable (111), apte à transmettre les ondes élastiques basse fréquence dudit générateur de vibration (102) audit transducteur ultrasonore (104).

2. Dispositif selon la revendication précédente **caractérisé en ce que** ledit dispositif (110) comporte un support (120) pour maintenir spatialement le transducteur ultrasonore (104).

3. Dispositif selon l'une au moins des revendications précédentes **caractérisé en ce qu'**il comporte un système ressort (115) situé entre ledit transducteur ultrasonore (104) et ledit tube déformable (111).

4. Dispositif selon l'une au moins des revendications précédentes **caractérisé en ce que** ledit dispositif (110) comporte un tube de préhension (117).

5. Dispositif selon l'une au moins des revendications précédentes **caractérisé en ce que** ledit générateur de vibration (102) est formé par une table vibrante (107).

6. Dispositif selon les revendications 1 à 4 **caractérisé en ce que** ledit générateur de vibration (102) est formé par un dispositif annexe de type haut-parleur.

7. Dispositif selon l'une au moins des revendications précédentes **caractérisé en ce que** ledit dispositif (110) comprend un système de réglage de précision (112) pour ajuster en rotation et/ou en translation la position dudit transducteur ultrasonore.

8. Dispositif selon l'une au moins des revendications précédentes **caractérisé en ce que** ledit générateur de vibration (102) se situe à l'extrémité opposée du tube déformable (111) où se situe le transducteur ultrasonore (104).

9. Dispositif selon l'une au moins des revendications précédentes **caractérisé en ce que** ledit dispositif (110) comporte au moins une barrette ultrasonore d'imagerie haute fréquence (116).

10. Dispositif selon l'une au moins des revendications précédentes **caractérisé en ce que** ledit dispositif (110) est apte à être couplé avec un dispositif de synchronisation organique (103).

11. Dispositif selon l'une au moins des revendications précédentes **caractérisé en ce que** ledit transducteur ultrasonore (104) possède un diamètre actif inférieur à 3 mm.

12. Procédé d'élastographie de mesures quantitatives et/ou qualitatives de propriétés viscoélastiques de tout milieu en utilisant un dispositif conforme à l'invention selon l'une quelconque des revendications précédentes ; ledit procédé comprend au moins les étapes suivantes:
- positionner, en déformant ledit tube déformable (111), perpendiculairement et au contact du milieu ledit au moins un transducteur ultrasonore (104) ;
- ajuster la position dudit transducteur ultrasonore (104) en translation et/ou en rotation ;
- déclencher au moins une mesure ;
- maintenir en position et au contact ledit transducteur ultrasonore (104) pendant au moins une mesure ;
ladite mesure comprend au moins les étapes suivantes :
- générer au moins une onde élastique basse fréquence ;
- émettre et acquérir simultanément à la génération d'au moins une onde élastique basse fréquence des ultrasons haute fréquence à l'aide dudit transducteur ultrasonore (104) pour observer la propagation de ladite au moins une onde élastique basse fréquence ;
- calculer au moins un paramètre relatif aux propriétés viscoélastiques du milieu tel que les variations des déplacements et/ou des déformations et/ou des vitesses de déplacement et/ou des vitesses de déformations engendrés dans le milieu ou plus généralement tout paramètre relatif aux propriétés viscoélastiques ;
- calculer les propriétés viscoélastiques du milieu.

13. Procédé selon la revendication précédente **caractérisé en ce que** ledit transducteur ultrasonore (102) est maintenu, par ledit tube déformable (111), en position et au contact du milieu pendant une série de mesures.

14. Procédé selon l'une des revendications 12 à 13 **caractérisé en ce que** lesdites ondes élastiques basse fréquence sont déclenchées par l'utilisateur de façon aléatoire débutant de façon synchronisée avec un rythme respiratoire et/ou cardiaque.

15. Procédé selon l'une des revendications 12 à 14 **caractérisé en ce que** ladite émission ultrasonore haute fréquence est réalisée sur une gamme de fréquence comprise entre 8 MHz et 100 MHz, et de préférence entre 10 MHz et 50 MHz.

16. Procédé selon l'une des revendications 12 à 15 **caractérisé en ce que** lesdites ondes élastiques basse fréquence sont générées sur une gamme de fréquence comprise entre 50 Hz et 10000 Hz.

17. Procédé selon l'une des revendications 12 à 15 **caractérisé en ce que** lesdites ondes élastiques basse fréquence sont de l'ordre de 400 Hz.

18. Procédé selon l'une des revendications 12 à 17 **caractérisé en ce que** ladite au moins une onde élastique basse fréquence est générée par vibration mécanique, et/ou par compression mécanique et/ou par focalisation ultrasonore.

## Patentansprüche

1. Elastographievorrichtung (110) für quantitative und / oder qualitative Messungen der viskoelastischen Eigenschaften jedes Milieus, wobei die genannte Vorrichtung (110) umfasst:
- einen Vibrationsgenerator (102) zum Erzeugen wenigstens einer elastischen Niedrigfrequenzwelle;
- einen Ultraschallwandler (104) zum Beobachten der Verbreitung der genannten wenigstens einen elastischen Niedrigfrequenzwelle;
- einen Auslöser (105) zum Auslösen wenigstens einer Messung;
wobei die genannte wenigstens eine Messung die folgenden Schritte umfasst:
- Erzeugen wenigstens einer elastischen Niedrigfrequenzwelle in einem Milieu;
- Gleichzeitig mit dem Erzeugen wenigstens einer elastischen Niedrigfrequenzwelle Ausgeben und Erwerben von Hochfrequenzultraschall mithilfe des genannten Ultraschallwandlers (104), um das Verbreiten der genannten wenigstens einen Niedrigfrequenzwelle zu beobachten;
wobei die genannte Vorrichtung ebenfalls umfasst:
• eine verformbare Röhre (111), die für ein Halten und eine Positionierung des genannten Ultraschallwandlers (104) zwischen dem genannten Vibrationsgenerator (102) und dem genannten Ultraschallwandler (104) positioniert ist, der am Ende der genannten verformbaren Röhre (111) derart angeordnet ist, dass während wenigstens einer Messung die Rechtwinkligkeit und der Kontakt zwischen dem genannten Ultraschallwandler (104) und dem Milieu gewährleistet ist;
• wenigstens ein im Innern der verformbaren Röhre (111) angeordneter Stift (121), der geeignet ist, um die elastischen Niedrigfrequenzwellen des genannten Vibrationsgenerators (102) auf den genannten Ultraschallwandler zu übertragen.

2. Vorrichtung gemäß dem voranstehenden Anspruch, **dadurch gekennzeichnet, dass** die genannte Vorrichtung (110) einen Sockel (120) zum räumlichen Halten des Ultraschallwandlers (104) umfasst.

3. Vorrichtung gemäß wenigstens einem der voranstehenden Ansprüche, **dadurch gekennzeichnet, dass** sie ein Federsystem (115) umfasst, das zwischen dem genannten Ultraschallwandler (104) und der genannten verformbaren Röhre (111) angeordnet ist.

4. Vorrichtung gemäß wenigstens einem der voranstehenden Ansprüche, **dadurch gekennzeichnet, dass** die genannte Vorrichtung (110) eine Greifröhre (117) umfasst.

5. Vorrichtung gemäß wenigstens einem der voranstehenden Ansprüche, **dadurch gekennzeichnet, dass** der genannte Vibrationsgenerator (102) durch einen Rütteltisch (107) geformt ist.

6. Vorrichtung gemäß den Ansprüchen 1 bis 4, **dadurch gekennzeichnet, dass** der genannte Vibrationsgenerator (102) durch eine Nebenvorrichtung vom Typ Lautsprecher geformt ist.

7. Vorrichtung gemäß wenigstens einem der voranstehenden Ansprüche, **dadurch gekennzeichnet, dass** die genannte Vorrichtung (110) ein System zur Präzisionseinstellung (112) zum Einstellen der Position des genannten Ultraschallwandlers in Rotation und / oder in Translation umfasst.

8. Vorrichtung gemäß irgendeinem der voranstehenden Ansprüche, **dadurch gekennzeichnet, dass** der genannte Vibrationsgenerator (102) am entgegengesetzten Ende der verformbaren Röhre (111) angeordnet ist, an dem der Ultraschallwandler (104) angeordnet ist.

9. Vorrichtung gemäß wenigstens einem der voranstehenden Ansprüche, **dadurch gekennzeichnet, dass** die genannte Vorrichtung (110) wenigstens eine Ultraschallklemmleiste für Hochfrequenzbildgebung (116) umfasst.

10. Vorrichtung gemäß wenigstens einem der voranstehenden Ansprüche, **dadurch gekennzeichnet, dass** die genannte Vorrichtung (110) geeignet ist, um mit einer organischen Synchronisationsvorrichtung (103) gekoppelt zu sein.

11. Vorrichtung gemäß wenigstens einem der voranstehenden Ansprüche, **dadurch gekennzeichnet, dass** der genannte Ultraschallwandler (104) einen aktiven Durchmesser von weniger als 3 mm besitzt.

12. Elastographieverfahren für quantitative und / oder qualitative Messungen der viskoelastischen Eigenschaften jedes Milieus per Verwenden einer erfindungsgemäßen Vorrichtung gemäß irgendeinem der voranstehenden Ansprüche; wobei das genannte Verfahren wenigstens die folgenden Schritte umfasst:
- per Verformen der genannten verformbaren Röhre (111) lotrechtes und kontaktierendes Positionieren des Milieus des wenigstens eines Ultraschallwandlers (104);
- Anpassen der Position des genannten Ultraschallwandlers (104) in Translation und / oder in Rotation;
- Auslösen wenigstens einer Messung;
- Halten des genannten Ultraschallwandlers (104) in Position und kontaktierend während wenigstens einer Messung;
wobei die genannte Messung wenigstens die folgenden Schritte umfasst:
- Erzeugen wenigstens einer elastischen Niedrigfrequenzelle;
- gleichzeitig zum Erzeugen wenigstens einer elastischen Niedrigfrequenzwelle Ausgeben und Erwerben der Hochfrequenz-Ultraschallwellen mithilfe des genannten Ultraschallwandlers (104) zum Beobachten der Verbreitung der genannten wenigstens einen elastischen Niedrigfrequenzwelle;
- Berechnen wenigstens eines Parameters bezüglich der viskoelastischen Eigenschaften des Milieus, wie z. B. die Variationen der Verschiebungen und / oder Verformungen und / oder der Verschiebungsgeschwindigkeiten und / der Verformungsgeschwindigkeiten, die in dem Milieu erzeugt sind, oder ganz allgemein jeder Parameter bezüglich der viskoelastischen Eigenschaften;
- Berechnen der viskoelastischen Eigenschaften des Milieus.

13. Verfahren gemäß dem voranstehenden Anspruch, **dadurch gekennzeichnet, dass** der genannte Ultraschallwandler (102) durch die genannte verformbare Röhre (111) während einer Serie von Messungen in Position und kontaktierend gehalten ist.

14. Verfahren gemäß einem der Ansprüche 12 bis 13, **dadurch gekennzeichnet, dass** die genannten elastischen Niedrigfrequenzwellen durch den Nutzer zufällig ausgelöst sind und synchronisiert mit einem Atem- und / oder Herzrhythmus beginnen.

15. Verfahren gemäß einem der Ansprüche 12 bis 14, **dadurch gekennzeichnet, dass** die genannte Hochfrequenzultraschallausgabe über einen Frequenzbereich realisiert ist, der zwischen 8 MHz und 100 MHz und bevorzugt zwischen 10 MHz und 50 MHz inbegriffen ist.

16. Verfahren gemäß einem der Ansprüche 12 bis 15, **dadurch gekennzeichnet, dass** die genannten elastischen Niedrigfrequenzwellen über einen Frequenzbereich erzeugt sind, der zwischen 50 Hz und 10.000 Hz inbegriffen ist.

17. Verfahren gemäß einem der Ansprüche 12 bis 15, **dadurch gekennzeichnet, dass** die genannten elastischen Niedrigfrequenzwellen in der Größenordnung von 400 Hz liegt.

18. Verfahren gemäß einem der Ansprüche 12 bis 17, **dadurch gekennzeichnet, dass** die genannte wenigstens eine elastische Niedrigfrequenzwelle per mechanischer Vibration und / oder per mechanischer Komprimierung und / oder per Ultraschallfokalisierung erzeugt ist.

## Claims

1. Elastography device (110) for quantitatively and/or qualitatively measuring viscoelastic properties of any medium, said device (110) comprising:
- a vibration generator (102) for generating at least one low-frequency elastic wave;
- an ultrasonic transducer (104) in order to observe the propagation of said at least one low-frequency elastic wave;
- a trigger (105) for triggering at least one measurement;
said at least one measurement comprising the following steps:
- generating at least one low-frequency elastic wave in a medium;
- emitting and acquiring simultaneously to the generating of at least one low-frequency elastic wave high-frequency ultrasounds using said ultrasonic transducer (104) in order to observe the propagation of said at least one low-frequency elastic wave;
said device also comprising:
• a deformable tube (111), positioned between said vibration generator (102) and said ultrasonic transducer (104), for a maintaining and a positioning of said ultrasonic transducer (104) located at the end of said deformable tube (111) in such a way as to ensure, for at least one measurement, the perpendicularity and the contact between said ultrasonic transducer (104) and the medium;
• at least one rod (121) located inside the deformable tube (11), able to transmit the low-frequency elastic waves from said vibration generator (102) to said ultrasonic transducer (104).

2. Device according to the preceding claim **characterised in that** said device (110) comprises a support (120) in order to spatially maintain the ultrasonic transducer (104).

3. Device according to at least one of the preceding claims **characterised in that** it comprises a spring system (115) located between said ultrasonic transducer (104) and said deformable tube (111).

4. Device according to at least one of the preceding claims **characterised in that** said device (110) comprises a gripper tube (117).

5. Device according to at least one of the preceding claims **characterised in that** said vibration generator (102) is formed by a vibration table (107).

6. Device according to claims 1 to 4 **characterised in that** said vibration generator (102) is formed by an annex device of the speaker type.

7. Device according to at least one of the preceding claims **characterised in that** said device (110) comprises a precision adjustment system (112) in order to adjust in rotation and/or in translation the position of said ultrasonic transducer.

8. Device according to at least one of the preceding claims **characterised in that** said vibration generator (102) is located at the opposite end of the deformable tube (111) where the ultrasonic transducer (104) is located.

9. Device according to at least one of the preceding claims **characterised in that** said device (110) comprises at least one ultrasonic array of high-frequency imaging (116).

10. Device according to at least one of the preceding claims **characterised in that** said device (110) is able to be coupled with a device for organic synchronisation (103).

11. Device according to at least one of the preceding claims **characterised in that** said ultrasonic transducer (104) has an active diameter less than 3 mm.

12. Elastography method for quantitatively and/or qualitatively measuring viscoelastic properties of any medium by using a device in accordance with the invention according to any preceding claim; said method comprising at least the following steps:
- positioning, by deforming said deformable tube (111), perpendicularly and in contact with the medium said at least one ultrasonic transducer (104);
- adjusting the position of said ultrasonic transducer (104) in translation and/or in rotation;
- triggering at least one measurement;
- maintaining in position and in contact said ultrasonic transducer (104) for at least one measurement;
said measurement comprises at least the following steps:
- generating at least one low-frequency elastic wave;
- emitting and acquiring simultaneously to the generating of at least one low-frequency elastic wave high-frequency ultrasounds using said ultrasonic transducer (104) in order to observe the propagation of said at least one low-frequency elastic wave;
- calculating at least one parameter relative to the viscoelastic properties of the medium such as the variations of the displacements and/or of the deformations and/or displacement speeds and/or deformation speeds generated in the medium or more generally any parameter relative to the viscoelastic properties;
- calculating the viscoelastic properties of the medium.

13. Method according to the preceding claim **characterised in that** said ultrasonic transducer (102) is maintained, by said deformable tube (111), in position and in contact with the medium for a series of measurements.

14. Method according to one of claims 12 to 13 **characterised in that** said low-frequency elastic waves are triggered by the user randomly starting in a synchronised manner with a respiratory and/or heart rate rhythm.

15. Method according to one of claims 12 to 14 **characterised in that** said high-frequency ultrasonic emission is carried out over a frequency range between 8 MHz and 100 MHz, and preferably between 10 MHz and 50 MHz.

16. Method according to one of claims 12 to 15 **characterised in that** said low-frequency elastic waves are generated over a frequency range between 50 Hz and 10,000 Hz.

17. Method according to one of claims 12 to 15 **characterised in that** said low-frequency elastic waves are of about 400 Hz.

18. Method according to one of claims 12 to 17 **characterised in that** said at least one low-frequency elastic wave is generated by mechanical vibration, and/or by mechanical compression and/or by ultrasonic focalisation.
